(19) Europäisches Patentamt
European Patent Office
Office européen des brevets

(11) **EP 2 934 747 B1**

(12) **FASCICULE DE BREVET EUROPEEN**

(45) Date de publication et mention de la délivrance du brevet:
**24.05.2017 Bulletin 2017/21**

(21) Numéro de dépôt: **13812012.6**

(22) Date de dépôt: **04.12.2013**

(51) Int Cl.:
*B01J 29/72* [(2006.01)]    *B01J 29/74* [(2006.01)]
*B01J 29/76* [(2006.01)]    *B01J 35/10* [(2006.01)]
*B01J 37/02* [(2006.01)]    *B01J 37/00* [(2006.01)]
*B01J 37/10* [(2006.01)]    *C07C 15/08* [(2006.01)]
*C07C 5/27* [(2006.01)]

(86) Numéro de dépôt international:
**PCT/FR2013/052942**

(87) Numéro de publication internationale:
**WO 2014/096606 (26.06.2014 Gazette 2014/26)**

(54) **CATALYSEUR MODIFIE DE TYPE STRUCTURAL MTW, SA MÉTHODE DE PRÉPARATION ET SON UTILISATION DANS UN PROCÉDÉ D'ISOMÉRISATION D'UNE COUPE C8 AROMATIQUE**

MODIFIZIERTER KATALYSATOR MIT MTW-STRUKTUR, DESSEN HERSTELLUNGSMETHODE UND ANWENDUNG ZUR ISOMERISIERUNG VON C8 AROMATEN

MODIFIED CATALYST OF MTW-STRUCTURAL TYPE, PREPARATION METHOD AND USE IN A PROCESS OF ISOMERISATION OF C8 AROMATIC FEED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.12.2012 FR 1203535**

(43) Date de publication de la demande:
**28.10.2015 Bulletin 2015/44**

(73) Titulaire: **IFP Énergies nouvelles**
**92852 Rueil-Malmaison Cedex (FR)**

(72) Inventeurs:
• **GUILLON, Emmanuelle**
**F-69390 Vourles (FR)**
• **BRANDHORST, Laure**
**F-69008 Lyon (FR)**

(56) Documents cités:
**EP-A1- 2 047 906**    **WO-A1-2009/076024**
**WO-A1-2010/000652**    **WO-A1-2012/066012**
**US-A- 5 763 720**

EP 2 934 747 B1

**Description**

**Domaine de l'invention**

L'invention concerne la préparation du catalyseur selon l'invention.

**État de la technique antérieure**

L'isomérisation de la coupe C8 aromatique (composés aromatiques à 8 atomes de carbone par molécule) est la principale voie de formation du paraxylène, produit très recherché dans l'industrie pétrochimique, utilisé notamment pour la fabrication de fibres et films polyester. La coupe C8 aromatique issue du reformage catalytique ou du vapocraquage comporte le méta, para, ortho-xylène et l'éthylbenzène. Le coût de la séparation par distillation de l'éthylbenzène étant trop élevé, seuls le paraxylène et éventuellement l'ortho-xylène sont séparés, par séparation sélective sur zéolithes selon différents procédés de séparation. La coupe résiduelle C8 comprenant l'éthylbenzène est alors transformée dans une unité d'isomérisation, l'objectif étant de maximiser la fraction de paraxylène et de transformer l'éthylbenzène en xylènes ou en benzène.

L'isomérisation des xylènes se produit suivant un mécanisme monofonctionnel acide, la fonction acide étant généralement apportée par une zéolithe. En revanche, la transformation de l'éthylbenzène nécessite un catalyseur bifonctionnel présentant à la fois une fonction acide et une fonction hydrogénante. Dans les procédés existants, l'éthylbenzène est soit isomérisé en xylènes, soit désalkylé en benzène. On parle alors soit d'isomérisation isomérisante, soit d'isomérisation désalkylante. Les catalyseurs employés sont généralement des catalyseurs bifonctionnels alliant une phase zéolithique, au moins un métal et un liant (encore appelée matrice).

Parmi les zéolithes utilisées en isomérisation des coupes C8 aromatiques, on trouve la ZSM-12, une zéolithe de type structural MTW. Ces catalyseurs sont notamment décrits dans les demandes de brevets WO 2010/000652 A1 et WO 2012/066012 A1. Une amélioration des catalyseurs zéolithiques en isomérisation isomérisante en xylènes consisterait à augmenter la conversion de l'éthylbenzène, étape la plus difficile de cette transformation. En particulier, des réactions parasites secondaires telles que la dismutation et la désalkylation de l'éthylbenzène limitent la valorisation de ce composé en xylènes.

Une voie d'amélioration consiste à modifier le catalyseur par différents traitements ce qui peut engendrer des changements de caractéristiques de la zéolithe et/ou du liant utilisés. Un traitement particulier est le traitement à la vapeur d'eau (ou steaming selon la terminologie anglo-saxonne).

Le brevet US 4,784,747 décrit des catalyseurs ayant subi un traitement à la vapeur, ce qui permet d'augmenter l'activité catalytique en particulier en craquage et déparaffinage. Les meilleures performances ont été obtenues pour un traitement à la vapeur d'eau entre 200 et 500°C.

Le brevet EP 034 444 B2, décrit des catalyseurs, notamment la ZSM-5, ayant subi un traitement à la vapeur à une pression partielle d'eau inférieure à 100 mbar (1 bar = 0,1 MPa), pour l'augmentation de leur activité en craquage du n-hexane.

La demanderesse a découvert qu'un procédé de préparation selon la revendication 1 d'un catalyseur zéolithique comprenant au moins la zéolithe ZSM-12 ayant subi un traitement particulier en présence de vapeur d'eau présentait une forte amélioration de la conversion en éthylbenzène et l'approche à l'équilibre thermodynamique du paraxylène lors de son utilisation dans un procédé d'isomérisation isomérisante d'une coupe C8 aromatique comprenant au moins de l'éthylbenzène.

**Description détaillée de l'invention**

**Le catalyseur**

Le catalyseur obtenu selon le procédé de l'invention comprend au moins une zéolithe de type structural MTW, une matrice, au moins un métal du groupe VIII de la classification périodique des éléments (correspondant aux groupes 8 à 10 de la nouvelle classification périodique des éléments, CRC Handbook of Chemistry and Physics, 200-2001), ledit catalyseur ayant un volume mésoporeux augmenté d'au moins 10%, de préférence d'au moins 10,5% par rapport à son volume mésoporeux initial (compris généralement entre 0,55 et 0,75 mL/g) à l'issue d'un traitement avec de la vapeur d'eau à une pression partielle comprise entre 0,04 et 0,06 MPa et à une température comprise entre 300 et 400°C pendant au moins 0,5 heure.

Le volume mésoporeux du catalyseur est obtenu par soustraction du volume microporeux au volume poreux du catalyseur. Le volume microporeux et le volume poreux sont déterminés à partir de l'isotherme d'adsorption d'azote respectivement par la méthode t et par le volume d'azote adsorbé (mL/g) à une pression relative de 0,95, selon le livre Adsorption by Powders, F.Rouquerol et al., Academic Press 1999.

Il a été constaté que le traitement du catalyseur avec de la vapeur d'eau dans des conditions contrôlées particulières, c'est-à-dire à une pression partielle comprise entre 0,04 et 0,06 MPa et à une température comprise entre 300 et 400°C, pendant au moins 0,5 heure, permet d'augmenter la conversion en éthylbenzène et l'approche à l'équilibre thermodynamique du paraxylène lors de l'utilisation du catalyseur traité dans un procédé d'isomérisation isomérisante d'une coupe C8 aromatique contenant au moins de l'éthylbenzène.

Sans vouloir être lié par une quelconque théorie, cette augmentation de la conversion pourrait être liée en partie à l'augmentation du volume mésoporeux du catalyseur résultant du traitement à la vapeur d'eau tel que décrit ci-dessus.

Le traitement à la vapeur est réalisé avec une pression partielle de vapeur d'eau entre 0,04 et 0,06 MPa, dilué de préférence dans l'air.

Avantageusement, le traitement à la vapeur est réalisé à une température comprise entre 300 et 380°C pendant 0,5 heure et 24 heures, de préférence entre 1 heure et 12 heures.

La zéolithe de type structural MTW utilisée est la zéolithe ZSM-12.

La zéolithe ZSM-12 est une zéolithe bien connue ayant une structure à base d'aluminosilicate et pouvant éventuellement inclure un ou plusieurs autres éléments. De nombreuses méthodes d'obtention de cette zéolithe sont connues et sont disponibles dans l'art antérieur. Une définition de la ZSM-12 est donnée dans la "Database of zeolite structures" publiée en 2007/2008 par la "Structure Comission of the International Zeolite Association".

Avantageusement, la teneur en zéolithe est comprise entre 1 et 20% poids par rapport à la masse du support, le support correspondant au mélange de la zéolithe et de la matrice.

Le catalyseur est de préférence composé :

- de 1 à 20% bornes incluses, de préférence de 1 à 10% bornes incluses en poids d'au moins une zéolithe de type structural MTW comprenant du silicium et au moins un élément T choisi dans le groupe formé par l'aluminium, le fer, le gallium et le bore, avec un rapport atomique Si/T compris entre 20 et 200 bornes incluses et de préférence entre 20 et 100 bornes incluses. De préférence, ledit élément T est choisi dans le groupe constitué par l'aluminium et le bore, de manière plus préféré l'élément T est l'aluminium,
- de 0,01 à 2% bornes incluses et de préférence de 0,05 à 1% bornes incluses en poids, d'au moins un métal du groupe VIII de la classification périodique des éléments, ledit métal du groupe VIII étant déposé sur la zéolithe ou sur le liant,
- le complément à 100 % en poids d'au moins une matrice.

Le rapport atomique Si/Al global, déterminé par fluorescence X ou absorption atomique, prend en compte aussi bien les atomes d'aluminium présents dans la charpente zéolithique que les atomes d'aluminium éventuellement présents hors de ladite charpente zéolithique encore appelés aluminium extra-réseau. Avantageusement, la zéolithe de type structural MTW utilisée a un rapport Si/Al compris entre 20 et 200 bornes incluses, de préférence entre 20 et 100 bornes incluses.

### Procédé de préparation du catalyseur

La présente invention concerne un procédé de préparation du catalyseur selon l'invention.

Le catalyseur selon l'invention est préparé selon un procédé comprenant les étapes suivantes :

i) on utilise au moins la zéolithe ZSM-12,
ii) on prépare un support par mise en forme de ladite zéolithe avec une matrice,
iii) on dépose au moins un métal du groupe VIII de la classification périodique des éléments sur ledit support ou sur ladite zéolithe, l'ordre de réalisation desdites étapes ii) et iii) étant indifférent à la suite de ladite étape i),
iv) on met en contact le catalyseur issue de l'étape ii) ou de l'étape iii) selon l'ordre de leur réalisation, avec de la vapeur d'eau à une pression partielle comprise entre 0,04 et 0,06 MPa, à une température comprise entre 300 et 400°C, pendant au moins 0,5 heure, de telle sorte que le volume mésoporeux du catalyseur soit augmenté d'au moins 10%, par rapport au volume mésoporeux initial du catalyseur compris généralement entre 0,55 et 0,75 mL/g.

De préférence, la vapeur d'eau utilisée est diluée dans un gaz neutre, du dioxygène ou dans l'air.

Avantageusement, la zéolithe ZSM-12 utilisée à l'étape i) a un rapport Si/Al compris entre 20 et 200 bornes incluses, de préférence entre 20 et 100 bornes incluses.

Avantageusement, la zéolithe de ZSM-12 est mise en forme à l'étape ii) avec une matrice avec une teneur en zéolithe entre 1 et 20% poids, de préférence entre 1 et 10% poids par rapport à la masse du support.

Le traitement à la vapeur de l'étape iv) est réalisé avec une pression partielle de vapeur d'eau comprise entre 0,04 et 0,06 MPa, diluée de préférence dans l'air.

Avantageusement, le traitement à la vapeur de l'étape iv) est réalisé à une température comprise entre 300 et 380°C pendant 0,5 heure à 24 heures, de préférence entre 1 heure à 12 heures.

Avantageusement, le débit de gaz formé de vapeur d'eau est compris entre 0,2 et 10 L/h/g (Litres par heure par gramme) de support zéolithique.

### Échange ionique

Ladite zéolithe ZSM-12 que comprend le catalyseur est au moins en partie sous forme acide, c'est à dire sous forme hydrogène (H); le cation compétiteur C étant choisi dans le groupe constitué par les cations alcalins ou alcalino-terreux, de préférence dans le groupe constitué par les cations $Na^+$ et $K^+$, de préférence le cation compétiteur C est le cation $Na^+$.

Un échange ionique, précédé d'une calcination, peut être réalisé après l'étape i) en présence de nitrate d'ammonium ou d'acétate d'ammonium à une concentration de 0,005 à 15 N, de préférence 0,1 à 10 N, à une température comprise entre 15 et 100°C, pendant une durée de 1 à 10 heures dans un réacteur batch ou continu. En général après l'étape d'échange, la zéolithe obtenue est séchée par exemple à l'étuve, à une température comprise entre la température ambiante et 250°C, avant d'être calcinée à une température comprise entre 300°C et 600°C sous air. Il est possible de procéder à des échanges successifs. Le ou les échange(s) peut(vent) être réalisé(s) sur la zéolithe du support après l'étape ii).

### Préparation du support

L'étape de mise en forme (étape ii)) est généralement telle que le catalyseur est de préférence sous forme d'extrudés ou de billes en vue de son utilisation. Dans une variante de la préparation du catalyseur, la mise en forme est réalisée avant la calcination et l'échange ionique.

Pour la mise en oeuvre de ladite étape ii) de préparation du support la matrice est une alumine.

La teneur en zéolithe dans le support est comprise entre 1 et 20% poids, de préférence entre 1 et 10% poids.

### Séchage et calcination du support

La préparation du support conformément à ladite étape ii) est avantageusement suivie d'un séchage puis d'une calcination. Le séchage est de préférence réalisé à une température comprise entre 100 et 150°C pendant une durée comprise entre 5 et 20 heures en étuve. La calcination est de préférence réalisée à une température comprise entre 250°C et 600°C pendant une durée comprise entre 1 et 8 heures.

### Dépôt du métal

Conformément à l'invention, l'étape iii) de préparation du catalyseur comprenant la zéolithe ZSM-12 consiste à déposer au moins un métal du groupe VIII de la classification périodique des éléments et éventuellement au moins un métal choisi parmi les métaux des groupes IIIA, IVA et VIIB.

Ledit métal du groupe VIII présent dans le catalyseur selon l'invention est choisi parmi le fer, le cobalt, le nickel, le ruthénium, le rhodium, le palladium, l'osmium, l'iridium et le platine, de préférence parmi les métaux nobles et de manière plus préférée parmi le palladium et le platine. De manière encore plus préférée, ledit métal du groupe VIII est le platine. Selon la méthode mise en oeuvre pour le dépôt dudit métal du groupe VIII, comme il est indiqué ci-après dans la description, ledit métal du groupe VIII, de préférence le platine, peut être déposé de manière prépondérante sur la zéolithe ou sur la matrice.

Le métal choisi parmi les métaux des groupes IIIA, IVA et VIIB et éventuellement présent dans le catalyseur selon l'invention est choisi parmi le gallium, l'indium, l'étain et le rhénium, de préférence parmi l'indium, l'étain et le rhénium.

La préparation du catalyseur selon l'invention peut être effectuée par toute méthode connue de l'homme du métier. De manière préférée, à la suite de la calcination réalisée à la fin de l'étape ii) sur le support, au moins un métal VIII est introduit sur le support, à savoir soit majoritairement sur la matrice, soit majoritairement sur la zéolithe soit encore sur l'ensemble zéolithe-matrice. Le dépôt dudit métal sur le support est avantageusement effectué par la technique d'imprégnation à sec ou la technique d'imprégnation par excès. Lorsque plusieurs métaux sont introduits, ceux-ci peuvent être introduits soit tous de la même façon soit par des techniques différentes.

Tous les précurseurs de métaux du groupe VIII conviennent pour le dépôt d'un ou de plusieurs métal(ux) du groupe VIII sur le support. En particulier, pour tout métal noble du groupe VIII, on peut utiliser des composés ammoniaqués ou des composés tels que par exemple le chloroplatinate d'ammonium, le dichlorure de platine dicarbonyle, l'acide hexahydroxyplatinique, le chlorure de palladium ou le nitrate de palladium. Le platine est généralement introduit sous forme d'acide hexachloroplatinique. L'introduction du métal noble du groupe VIII est de préférence effectuée par imprégnation à l'aide d'une solution aqueuse ou organique de l'un des composés métalliques cités ci-dessus. Parmi les solvants organiques utilisables, on peut citer les hydrocarbures paraffiniques, naphténiques ou aromatiques contenant par exemple de 6 à 12 atomes de carbone par molécule, et les composés organiques halogénés contenant par exemple de 1 à 12 atomes de carbone par molécule. On peut citer par exemple le n-heptane, le méthylcyclohexane, le toluène et le chloroforme. On peut aussi utiliser les mélanges de solvants.

Le contrôle de certains paramètres mis en oeuvre lors du dépôt, en particulier la nature du précurseur du (des) métal(ux) du groupe VIII utilisé(s), permet d'orienter le dépôt du(es)dit(s) métal(ux) majoritairement sur la matrice ou sur la zéolithe.

Ainsi, pour introduire le(s) métal(ux) du groupe VIII, préférentiellement le platine et/ou le palladium, majoritairement sur la matrice, on peut mettre en oeuvre un échange anionique avec de l'acide hexachloroplatinique et/ou de l'acide hexachloropalladique, en présence d'un agent compétiteur, par exemple de l'acide chlorhydrique, le dépôt étant en général suivi d'une calcination, par exemple à une température comprise entre 350 et 550°C et pendant une durée comprise entre 1 et 4 heures. Avec de tels précurseurs, le(s) métal(ux) du groupe VIII est(sont) déposé(s) majoritairement sur la matrice et le(s)dit(s) métal(ux) présente(nt) une bonne dispersion et une bonne répartition macroscopique à travers le grain de catalyseur.

On peut aussi envisager de déposer le(s) métal(ux) du groupe VIII, préférentiellement le platine et/ou le palladium, par échange cationique de manière à ce que le(s)dit(s) métal(ux) soi(en)t déposé(s) majoritairement sur la zéolithe. Ainsi, dans le cas du platine, le précurseur peut être par exemple choisi parmi :

les composés ammoniaqués tels que les sels de platine (II) tétrammines de formule $Pt(NH_3)_4X_2$, les sels de platine (IV) hexammines de formule $Pt(NH_3)_6X_4$ ; les sels de platine (IV) halogénopentammines de formule $(PtX(NH_3)_5)X_3$ ; les sels de platine N-tétrahalogénodiammines de formule $PtX_4(NH_3)_2$ ; et
les composés halogénés de formule $H(Pt(acac)_2X)$ ;
X étant un halogène choisi dans le groupe formé par le chlore, le fluor, le brome et l'iode, X étant de préférence le chlore, et "acac" représentant le groupe acétylacétonate (de formule brute $C_5H_7O_2$), dérivé de l'acétylacétone. Avec de tels précurseurs, le(s) métal(ux) du groupe VIII est(sont) déposé(s) majoritairement sur la zéolithe et le(s)dit(s) métal(ux) présente(nt) une bonne dispersion et une bonne répartition macroscopique à travers le grain de catalyseur.

L'imprégnation à sec du métal du groupe VIII sur le support conduit au dépôt dudit métal à la fois sur la matrice et sur la zéolithe.

Dans le cas où le catalyseur selon l'invention contient également au moins un métal choisi parmi les métaux des groupes IIIA, IVA et VIIB, toutes les techniques de dépôt d'un tel métal connues de l'homme du métier et tous les précurseurs de tels métaux peuvent convenir.

On peut ajouter le(s) métal(ux) du groupe VIII et celui(ceux) des groupes IIIA, IVA et VIIB, soit séparément soit simultanément dans au moins une étape unitaire. Lorsqu'au moins un métal des groupes IIIA, IVA et VIIB est ajouté séparément, il est préférable qu'il soit ajouté après le métal du groupe VIII.

Le métal additionnel choisi parmi les métaux des groupes IIIA, IVA et VIIB peut être introduit par l'intermédiaire de composés tels que par exemple les chlorures, les bromures et les nitrates des métaux des groupes IIIA, IVA et VIIB. Par exemple dans le cas de l'indium, on utilise avantageusement le nitrate ou le chlorure et dans le cas du rhénium, on utilise avantageusement l'acide perrhénique. Dans le cas de l'étain, les chlorures d'étain $SnCl_2$ et $SnCl_4$ sont préférés. Le métal additionnel choisi parmi les métaux des groupes IIIA, IVA et VIIB peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel choisi parmi les métaux des groupes IIIA, IVA et VIIB est introduit avant le métal du groupe VIII, le composé du métal IIIA, IVA et/ou VIIB utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.

De plus, des traitements intermédiaires tels que par exemple une calcination et/ou une réduction peuvent être appliqués entre les dépôts successifs des différents métaux.

Avantageusement, le dépôt de(s) métal(aux) est suivi par une calcination, habituellement à une température comprise entre 250°C et 600°C, pour une durée comprise entre 0,5 et 10 heures, de préférence précédée d'un séchage, par exemple à l'étuve, à une température allant de la température ambiante à 250°C, de préférence de 40°C à 200°C. Ladite étape de séchage est de préférence menée pendant la montée en température nécessaire pour effectuer ladite calcination.

Le(s) métal(ux) du groupe VIII, de préférence le platine, déposé(s) sur la zéolithe et/ou sur la matrice, représente(nt) de 0,01 à 2 %, de préférence de 0,05 à 1 %, en poids par rapport au poids de catalyseur. La matrice constitue le complément à 100 %.

Lorsque ledit catalyseur contient au moins un métal choisi parmi les métaux des groupes IIIA, IVA et VIIB, la teneur en celui-ci peut aller jusqu'à 2 % en poids par rapport au poids de catalyseur. Elle est alors avantageusement de 0,01 à 2 %, de préférence de 0,05 à 1 % en poids. Lorsque ledit catalyseur contient du soufre, la teneur en celui-ci peut être telle que le rapport du nombre d'atomes de soufre sur le nombre d'atomes de métal du groupe VIII déposés aille jusqu'à 2:1. Il est alors avantageusement de 0,5:1 à 2:1.

**Traitement à la vapeur**

Selon l'invention, ledit catalyseur est soumis après l'étape ii) ou après l'étape iii) à un traitement à la vapeur d'eau de sorte que son volume mésoporeux soit augmenté d'au moins 10%, de préférence d'au moins 10,5% par rapport à son volume mésoporeux initial, ledit volume mésoporeux initial étant compris généralement entre 0,55 et 0,75 mL/g. L'augmentation de la mésoporosité peut être supérieure à 15% poids. A cet effet, le traitement en présence de vapeur d'eau auquel est soumis le catalyseur après l'étape ii) ou après l'étape iii) est réalisé dans des conditions contrôlées, à savoir : à une température comprise entre 300 et 400°C, de préférence entre 300 et 380°C, de manière encore plus préférée entre 330 et 370 °C. La durée dudit traitement est d'au moins 0,5 heure, de préférence entre 0,5 heure et 24 heures, et de manière plus préférée entre 1 heure et 12 heures. La pression partielle en vapeur d'eau durant le traitement est entre 0,04 et 0,06 MPa. La vapeur d'eau est généralement diluée dans un gaz neutre, du dioxygène ou dans l'air, de préférence dans l'air.

Le débit de gaz formé de vapeur d'eau est avantageusement compris entre 0,2 L/h/g et 10 L/h/g de support zéolithique.

On peut mettre en oeuvre une réduction préalable du catalyseur *ex situ* ou *in situ*, sous courant d'hydrogène, par exemple à une température de 450°C à 600°C, pendant une durée de 0,5 à 4 heures, avant la mise en oeuvre du procédé d'isomérisation.

Dans le cas où le catalyseur ne contient pas de soufre, une réduction du métal sous hydrogène est avantageusement réalisée *in situ* avant injection de la charge.

Dans le cas où le catalyseur contient du soufre, le soufre est introduit sur le catalyseur mis en forme, calciné, contenant le ou les métaux cités précédemment, soit *in situ* avant la réaction catalytique, soit *ex situ.* La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration. La sulfuration s'effectue en présence d'hydrogène en utilisant tout agent sulfurant bien connu de l'homme de métier, tel que par exemple le sulfure de

diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

Le procédé d'isomérisation consiste à mettre en contact une coupe aromatique contenant au moins un composé aromatique ayant huit atomes de carbone par molécule avec au moins ledit catalyseur contenant au moins ladite zéolithe ZSM-12, ledit catalyseur ayant été préparé conformément à la mise en oeuvre de chacune desdites étapes i), ii), iii) et iv) décrites plus haut dans la présente description.

**Procédé d'isomérisation**

La charge est une coupe aromatique contenant au moins un composé aromatique ayant huit atomes de carbone par molécule et comprend avantageusement soit uniquement un mélange de xylènes, soit uniquement de l'éthylbenzène, soit un mélange de xylène(s) et d'éthylbenzène.

Le procédé d'isomérisation comprend la mise en contact de ladite coupe avec le catalyseur.

Le procédé d'isomérisation est avantageusement mis en oeuvre selon les conditions opératoires suivantes :

- une température de 300°C à 500°C, de préférence de 320°C à 450°C et de manière plus préférée de 340°C à 430°C ;
- une pression partielle d'hydrogène de 0,3 à 1,5 MPa, de préférence de 0,4 et 1,2 MPa et de manière encore préférée de 0,7 à 1,2 MPa ;
- une pression totale de 0,45 à 1,9 MPa, de préférence de 0,6 à 1,5 MPa ; et

- une vitesse spatiale d'alimentation, exprimée en kilogramme de charge introduite par kilogramme de catalyseur et par heure, de 0,25 à 30 h⁻¹, de préférence de 1 à 10 h⁻¹ et de manière encore préférée de 2 à 6 h⁻¹.

Les exemples suivants illustrent l'invention sans toutefois en limiter la portée.

## EXEMPLES

**Exemple 1 (conforme invention)** : Préparation du catalyseur A traité à la vapeur d'eau à 350°C, contenant la zéolithe ZSM-12 et 0,3 % poids de platine.

La matière première utilisée est une zéolithe ZSM-12 brute de synthèse, comprenant le structurant organique, du silicium et de l'aluminium, possédant un rapport atomique Si/Al égal à 60. Cette zéolithe ZSM-12 subit une calcination à 550°C sous flux d'air durant 6 heures.

La zéolithe ZSM-12 calcinée est ensuite mise en forme par extrusion avec un gel d'alumine de manière à obtenir, après séchage et calcination sous air sec, un support constitué d'extrudés de 1,4 mm de diamètre, qui contient en poids environ 4% de zéolithe ZSM-12 et environ 96% d'alumine. Le volume mésoporeux du support est déterminé à partir de l'isotherme d'adsorption à l'azote et vaut 0,73 mL/g. Le support ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous air à la température de 500°C pendant une heure.

Le solide est ensuite traité par de la vapeur d'eau avec une pression partielle de 0,05 MPa dans l'air à 2L/h/g à 350°C pendant 10 heures. Après traitement, le volume mésoporeux du catalyseur est déterminé à partir de l'isotherme d'adsorption à l'azote et vaut 0,81 mL/g.

Le catalyseur ainsi obtenu contient en poids 4% de zéolithe ZSM-12 en partie sous forme hydrogène (H), 95,7% d'alumine et 0,3% de platine. La dispersion du platine, mesurée par chimisorption d'oxygène, est de 80% environ.

**Exemple 2 (non conforme)** : Préparation du catalyseur B sans traitement à la vapeur d'eau contenant de la zéolithe ZSM-12 et 0,3 % poids de platine.

La matière première utilisée est une zéolithe ZSM-12 brute de synthèse, comprenant le structurant organique, du silicium et de l'aluminium, possédant un rapport atomique Si/Al égal à 60. Cette zéolithe ZSM-12 subit une calcination à 550°C sous flux d'air durant 6 heures.

La zéolithe ZSM-12 calcinée est ensuite mise en forme par extrusion avec un gel d'alumine de manière à obtenir, après séchage et calcination sous air sec, un support constitué d'extrudés de 1,4 mm de diamètre, qui contient en poids environ 4 % de zéolithe ZSM-12 et environ 96 % d'alumine. Le volume mésoporeux du support est déterminé à partir de l'isotherme d'adsorption à l'azote et vaut 0,73 mL/g.

Le support ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous air à la température de 500°C pendant une heure.

Le catalyseur ainsi obtenu contient en poids 4% de zéolithe ZSM-12 en partie sous forme hydrogène (H), 95,7% d'alumine et 0,3% de platine. La dispersion du platine, mesurée par chimisorption d'oxygène, est de 80% environ.

**Exemple 3 (non conforme)** : Préparation du catalyseur C traité à la vapeur d'eau à 550°C, contenant de la zéolithe ZSM-12 et 0,3 % poids de platine.

La matière première utilisée est une zéolithe ZSM-12 brute de synthèse, comprenant le structurant organique, du silicium et de l'aluminium, possédant un rapport atomique Si/Al égal à 60. Cette zéolithe ZSM-12 subit une calcination à 550°C sous flux d'air durant 6 heures.

La zéolithe ZSM-12 calcinée est ensuite mise en forme par extrusion avec un gel d'alumine de manière à obtenir, après séchage et calcination sous air sec, un support constitué d'extrudés de 1,4 mm de diamètre, qui contient en poids environ 4 % de zéolithe ZSM-12 et environ 96 % d'alumine. Le volume mésoporeux du support est déterminé à partir de l'isotherme d'adsorption à l'azote et vaut 0,73 mL/g.

Le support ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous air à la température de 500°C pendant une heure.

Le solide est ensuite traité par de la vapeur d'eau avec une pression partielle de 0,09 MPa dans l'air à 2 L/h/g à 550°C pendant 10 heures. Après traitement, le volume mésoporeux du catalyseur est déterminé à partir de l'isotherme d'adsorption à l'azote et vaut 0,75 ML/g.

Le catalyseur ainsi obtenu contient en poids 4% de zéolithe ZSM-12 en partie sous forme hydrogène (H), 95,7% d'alumine et 0,3% de platine. La dispersion du platine, mesurée par chimisorption d'oxygène, est de 80% environ.

**Exemple 4 :** Évaluation des propriétés catalytiques des catalyseurs A, B et C en isomérisation d'une coupe C8 aromatique selon le procédé de l'invention.

Les performances des catalyseurs A, B et C ont été évaluées en isomérisation d'une coupe C8 aromatique contenant principalement du méta-xylène, de l'ortho-xylène et de l'éthylbenzène. Les caractéristiques de la charge sont les suivantes :

| Charge | % poids |
|---|---|
| Ethylbenzène | 19 |
| Orthoxylène | 14,4 |
| Metaxylène | 60 |

Les catalyseurs sont maintenus pendant 3 heures à 480°C sous débit d'hydrogène pur puis la charge est injectée.

Les catalyseurs A, B et C sont mis en contact avec la charge, les conditions opératoires sont les suivantes :

Température = 385°C,
Pression totale = 1 MPa,
Pression partielle d'hydrogène (ppH$_2$)= 0,88 MPa,
vitesse spatiale (V.V.H) = 8 h$^{-1}$.

Les catalyseurs ont été comparés en termes d'activité (par l'approche à l'équilibre du paraxylène et par la conversion de l'éthylbenzène).

Pour le calcul de l'approche à l'équilibre du paraxylène (AEQ pX), la concentration en paraxylène (%pX) est exprimée par rapport aux trois isomères xylènes.

L'approche à l'équilibre du paraxylène (AEQ pX) est définie de la manière suivante :

$$AEQ\ pX\ (\%) = 100 \times (\%pX_{effluent} - \%pX_{charge}) / (\%pX_{équilibre} - \%pX_{charge})$$

où

$\%pX_{effluent}$ = concentration en paraxylènes dans l'effluent à l'issue de la réaction,
$\%pX_{charge}$ = concentration en paraxylènes présents initialement dans la charge,
$\%pX_{équilibre}$ = concentration en paraxylènes à l'équilibre.

La conversion de l'éthylbenzène Cv EB(%) est définie de la manière suivante :

$$Cv\ EB\ (\%) = 100 \times (\%EB_{charge} - \%EB_{effluent})/\%EB_{charge}$$

où

$\%EB_{charge}$ = concentration en éthylbenzène présent initialement dans la charge,
$\%EB_{effluent}$ = concentration en éthylbenzène présent dans l'effluent à l'issue de la réaction.

Les résultats obtenus à iso-conditions opératoires sont présentés dans le tableau 1.

Tableau 1 : Activité des catalyseurs A, B et C après 4000 minutes de réaction

| Activité (%) | Catalyseur A | Catalyseur B | Catalyseur C |
|---|---|---|---|
| AEQ pX | 91,1 | 82,0 | 77,4 |
| Cv EB | 31,0 | 17,5 | 14,7 |

Le catalyseur A traité à la vapeur d'eau à 350°C avec une pression partielle en eau de 0,05 MPa présente un gain important en conversion de l'éthylbenzène ainsi qu'une activité en approche à l'équilibre thermodynamique du paraxylène nettement augmentée par rapport aux catalyseurs n'ayant pas subi de traitement à la vapeur (catalyseur B) et ayant subi un traitement à la vapeur à une température supérieure à 400°C et à une pression partielle en eau supérieure à 0,07 MPa (catalyseur C).

**Revendications**

1. Procédé de préparation d'un catalyseur comprenant au moins les étapes suivantes :

   i) on utilise au moins la zéolithe ZSM-12,
   ii) on prépare un support par mise en forme de ladite zéolithe avec une alumine,
   iii) on dépose au moins un métal du groupe VIII de la classification périodique des éléments sur ledit support ou sur ladite zéolithe, l'ordre de réalisation desdites étapes ii) et iii) étant indifférent à la suite de ladite étape i),
   iv) on met en contact le catalyseur issu de l'étape ii) ou de l'étape iii) selon l'ordre de leur réalisation, avec de la vapeur d'eau à une pression partielle comprise entre 0,04 et 0,06 MPa, à une température comprise entre 300 et 400°C, pendant au moins 0,5 heure, de telle sorte que le volume mésoporeux du catalyseur augmente d'au moins 10% par rapport au volume mésoporeux initial du catalyseur compris généralement entre 0,55 et 0,75 mL/g.

2. Procédé de préparation selon la revendication 1 dans lequel l'étape ii) est suivie d'un séchage réalisé à une température comprise entre 100 et 150°C pendant une durée comprise entre 5 et 20 heures en étuve, puis d'une calcination réalisée à une température comprise entre 250°C et 600°C pendant une durée comprise entre 1 et 8 heures.

3. Procédé de préparation selon la revendication 1 ou 2 dans lequel, l'étape ii) de dépôt de(s) métal(aux) est suivi d'une calcination à une température comprise entre 250°C et 600°C, pendant une durée comprise entre 0,5 et 10 heures, précédée d'un séchage à une température allant de la température ambiante à 250°C.

4. Procédé selon les revendications 1 à 3 dans lequel la température mise en oeuvre dans l'étape iv) est comprise entre 300 et 380°C pendant 0,5 heure à 24 heures.

5. Procédé selon les revendications 1 à 4 dans lequel la zéolithe de type structural MTW utilisée à l'étape i) a un rapport Si/Al compris entre 20 et 200 bornes incluses, de préférence entre 20 et 100 bornes incluses.

6. Procédé de préparation selon les revendications 1 à 5 dans lequel le catalyseur est réduit *ex situ* ou *in situ* avant son utilisation, sous courant d'hydrogène à une température de 450°C à 600°C, pendant une durée de 0,5 à 4 heures.

**Patentansprüche**

1. Verfahren zur Herstellung eines Katalysators, das mindestens die folgenden Schritte umfasst:

   i) Verwenden mindestens des Zeolithen ZSM-12,
   ii) Herstellen eines Trägers durch Formen des Zeolithen mit einem Aluminiumoxid,
   iii) Abscheiden mindestens eines Metalls der Gruppe VIII des Periodensystems der Elemente auf dem Träger oder auf dem Zeolithen, wobei die Reihenfolge der Ausführung der Schritte ii) und iii) nach Schritt i) nicht von Bedeutung ist,
   iv) Inkontaktbringen des Katalysators aus Schritt ii) oder Schritt iii), abhängig von der Reihenfolge der Ausführung, mit Wasserdampf bei einem Partialdruck im Bereich zwischen 0,04 und 0,06 MPa, bei einer Temperatur im Bereich zwischen 300 und 400 °C für mindestens 0,5 Stunden, so dass sich das Mesoporenvolumen des Katalysators um mindestens 10 % erhöht, bezogen auf das Ausgangsmesoporenvolumen des Katalysators, das im Allgemeinen im Bereich zwischen 0,55 und 0,75 ml/g liegt.

2. Verfahren zur Herstellung nach Anspruch 1, wobei auf Schritt ii) eine Trocknung, die bei einer Temperatur im Bereich zwischen 100 und 150 °C für eine Dauer im Bereich zwischen 5 und 20 Stunden im Ofen durchgeführt wird, dann eine Kalzinierung folgt, die bei einer Temperatur im Bereich zwischen 250 °C und 600 °C für eine Dauer im Bereich zwischen 1 und 8 Stunden durchgeführt wird.

3. Verfahren zur Herstellung nach Anspruch 1 oder 2, wobei auf den Schritt ii) zum Abscheiden des Metalls (der Metalle) eine Kalzinierung bei einer Temperatur im Bereich zwischen 250 °C und 600 °C für eine Dauer im Bereich zwischen 0,5 und 10 Stunden folgt, der eine Trocknung bei einer Temperatur vorausgeht, die von der Umgebungstemperatur bis 250 °C reicht.

4. Verfahren nach den Ansprüchen 1 bis 3, wobei die Temperatur, die im Schritt iv) eingesetzt wird, im Bereich zwischen 300 und 380 °C für 0,5 Stunden bis 24 Stunden liegt.

5. Verfahren nach den Ansprüchen 1 bis 4, wobei der Zeolith mit MTW-Struktur, der im Schritt i) verwendet wird, ein Si/Al-Verhältnis im Bereich zwischen einschließlich 20 und einschließlich 200, bevorzugt zwischen einschließlich 20 und einschließlich 100, aufweist.

6. Verfahren zur Herstellung nach den Ansprüchen 1 bis 5, wobei der Katalysator ex situ oder in situ vor seiner Verwendung unter einem Wasserstoffstrom bei einer Temperatur von 450 °C bis 600 °C für eine Dauer von 0,5 bis 4 Stunden reduziert wird.

**Claims**

1. A process for the preparation of a catalyst comprising at least the following steps:

   i) using at least the ZSM-12 zeolite,
   ii) preparing a support by shaping said zeolite with an alumina,
   iii) depositing at least one metal from group VIII of the periodic classification of the elements onto said support or onto said zeolite, the order of carrying out said steps ii) and iii) being unimportant following said step i),
   iv) bringing the catalyst obtained in step ii) or step iii), depending on the order in which they are carried out, into contact with steam at a partial pressure in the range 0.04 to 0.06 MPa, at a temperature in the range 300°C to 400°C, for at least 0.5 hour, in a manner such that the mesopore volume of the catalyst is increased by at least 10% compared with the initial mesopore volume of the catalyst, which is generally in the range 0.55 to 0.75 mL/g.

2. The preparation process according to claim 1, in which step ii) is followed by drying carried out at a temperature in the range 100°C to 150°C for a period in the range 5 to 20 hours in an oven, then by calcining carried out at a temperature in the range 250°C to 600°C for a period in the range 1 to 8 hours.

3. The preparation process according to claim 1 or claim 2, in which step ii) for depositing the metal(s) is followed by calcining at a temperature in the range 250°C to 600°C, for a period in the range 0.5 to 10 hours, preceded by drying at a temperature from ambient temperature to 250°C.

4. Process according to one of claims 1 to 3 in which step iv) is carried out at a temperature in the range 300 to 380°C, for a period of 0.5 to 24 hours.

5. Process according to one of claims 1 to 4 in which the zeolite with structure type MTW used in step i) has a Si/Al ratio in the range 20 to 200, limits included, preferably in the range 20 to 100, limits included.

6. The preparation process according to one of claims 1 to 5, in which the catalyst is reduced *ex situ* or *in situ* before it is used, in a stream of hydrogen at a temperature of 450°C to 600°C, for a period of 0.5 to 4 hours.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010000652 A1
- WO 2012066012 A1
- US 4784747 A
- EP 034444 B2

**Littérature non-brevet citée dans la description**

- CRC Handbook of Chemistry and Physics. 2001, vol. 200
- **F.ROUQUEROL et al.** Adsorption by Powders. Academic Press, 1999